# EUROPÄISCHE PATENTSCHRIFT

(11) **EP 1 579 888 B1**
(45) Veröffentlichungstag und Bekanntmachung des Hinweises auf die Patenterteilung: **16.07.2014**
(21) Anmeldenummer: 05006502.8
(22) Anmeldetag: 24.03.2005
(51) Int. Cl.: A61M 39/22, A61M 39/14, A61M 39/18

(54) **Übergangsverschluss für Blutbeutelsysteme**
Coupling for bloodbagsystems
Raccord pour un ensemble de poches à sang

(30) Priorität: 27.03.2004 DE 102004015031
(43) Veröffentlichungstag der Anmeldung: 28.09.2005
(73) Patentinhaber: Schmitz, Frank, 48432 Rheine (DE)
(72) Erfinder: Schmitz, Frank, 48432 Rheine (DE)
(74) Vertreter: Lobemeier, Martin Landolf

(56) Entgegenhaltungen:
- GB-A- 2 060 399
- US-A- 5 456 675
- US-A- 5 534 228
- US-A- 6 146 374

## Beschreibung

Die Erfindung betrifft einen Übergangsverschluss für Blutbeutelsysteme, über den ein Blutbeutel-Anschluss, der einen bestimmten Lumen aufweist, mit einem Transferschlauch zu verbinden ist und der unter Sterilhaltung des Fließweges manuell zu öffnen ist. Bei einem für die direkte Aufnahme von Spenderblut bestimmten Blutbeutelsystem gemäß US 6 146 374 A und Oberbegriff des Anspruches 1 weist ein solches System folgende Teile auf:
- zwei in Axialrichtung hintereinander liegende und zueinander axial bewegliche Außenrohre, die an ihren entfernt liegenden Enden mit dem Blutbeutel-Anschluss bzw. dem Transferschlauch verbindbar sind,
- ein Innenrohr, das mit dem ersten Außenrohr endseitig überstehend verbunden ist, so dass es mit einem Überstand über die Stirnseite des Außenrohres übersteht, und axial verschiebbar in dem Lumen des zweiten Außenrohres eingeführt ist,
- ein Siegelelement, das den Fluss durch den Übergangsverschluss sperrt.

Für die Lagerung des entnommenen Humanbluts sind Blutbeutelsysteme bekannt, die von verschiedenen Herstellern angeboten werden. Von der Firma Fresenius HemoCare GmbH wird beispielsweise ein aus vier Blutbeuteln bestehendes System vertrieben, bei dem zunächst in einem Primärbeutel die humane Vollblut-Spende aufgenommen und gelagert wird. Aus dem Primärbeutel wird nach Zentrifugation je nach Arbeitsweise der betreffenden Blutbank ein Abzug vorgenommen, so dass das Blut sukzessive in Sekundärblutbeutel gelangt. Die Blutbeutel sind untereinander durch Transferschläuche verbunden, wobei zwischen dem jeweiligen Blutbeutel-Ausgang und dem Transferschlauch ein Übergangsverschluss vorhanden ist.

Bei dem System der Firma Fresenius HemoCare GmbH ist beispielsweise als Übergangsverschluss ein Abbruchventil vorgesehen, das unter sterilem Verschluss manuell geöffnet werden muss. Hierzu wird gemäß Anweisung der Herstellerin die Hülse des Abbruchventils mit der einen Hand nach unten und mit der anderen Hand nach oben gehalten. Die Spitze der Hülse wird mit einem Winkel von 90° nach hinten und wieder nach vorne gebrochen, wobei das Abbrechventil zunächst leicht geöffnet ist, aber noch nicht das Blut sukzessive in Sekundärblutbeutel gelangt. Die Blutbeutel sind untereinander durch Transferschläuche verbunden, wobei zwischen dem jeweiligen Blutbeutel-Ausgang und dem Transferschlauch ein Übergangsverschluss vorhanden ist.

Bei dem System der Firma Fresenius HemoCare GmbH ist beispielsweise als Übergangsverschluss ein Abbruchventil vorgesehen, das unter sterilem Verschluss manuell geöffnet werden muss. Hierzu wird gemäß Anweisung der Herstellerin die Hülse des Abbruchventils mit der einen Hand nach unten und mit der anderen Hand nach oben gehalten. Die Spitze der Hülse wird mit einem Winkel von 90° nach hinten und wieder nach vorne gebrochen, wobei das Abbrechventil zunächst leicht geöffnet ist, aber noch nicht korrekt positioniert ist. Anschließend werden erneut die Spitze der Hülse mit der Hand in einem Winkel von 90° nach hinten und wieder nach vorne geschoben. Dabei muss der Abstand zwischen dem Abbrechventilkegel und seiner Ausgangsposition mindestens 3 mm betragen. Außerdem muss sich die bedienende Person vergewissern, dass die Spitze des Abbrechventils den Schlauch nicht wieder verschließt.

Das manuelle Abbrechen muss mit einer erheblichen Kraft erfolgen, wobei keine Hilfsapparate eingesetzt werden können, sondern die Kraft allein von den haltenden Fingern und der Hand aufgebracht werden muss. Bei den Blutbanken, bei denen pro Blutbeutelsystem jeweils drei derartiger Übergangsverschlüsse geöffnet und pro Arbeitsschicht hunderte derartiger Blutbeutelsysteme geöffnet werden, kommt es zur Überbeanspruchung von Fingern und Gelenken. Weiterhin wird beobachtet, dass bei den Übergangsverschlüssen gemäß Stand der Technik sich an den Bruchstellen scharfe Kanten bilden können, so dass vorbeiströmende Erythrozyten geschädigt werden und in dem Präparat zu Hemolysen führen können.

Überdies ist die Einbringung einer Kanüle aus einem Fremdmaterial kompliziert und kostspielig.

In der US 6 146 374 wird ein Übergangsverschluss für medizinische Beutelsysteme, z.B. Katheter oder Infusionssysteme offenbart, bei dem beim Einschieben eines Innenrohrs in ein gegenüberliegendes Außenrohr ein dort vorhandenes Ventilelement aufgespreizt wird. Der Lumen des Außenrohrs ist deutlich größer als der Lumen des Innenrohrs, so dass der aufgespreizte Ventilabschnitt im Strömungsquerschnitt angeordnet ist. Dies führt zu einer vermehrten Berührung der Blutzellen mit den Kanten des aufgespreizten Ventilabschnitts und kann damit Hämolyse verursachen.

Es stellt sich demnach die Aufgabe, einen Übergangsverschluss speziell für zu entleerende Blutbeutelsysteme anzugeben, bei dem das Öffnen des Fließweges einfach ist und bei dem eine sichere Öffnung der Übergangsverschlüsse erfolgen kann, ohne dass übermäßige Aufmerksamkeit und Kraft erforderlich sind. Außerdem sollen scharfkantige Bereiche und eingesetzte Kanülen vermieden werden.

Diese Aufgabe wird gelöst bei einem Übergangsverschluss der eingangs genannten Art, der dadurch gekennzeichnet ist, dass zur Verbindung eines Blutbeutel-Anschlusses mit einem Transferschlauch der Blutbeutel-Anschluss von der einen Seite des ersten Außenrohres und von der gegenüberliegenden Seite ein Innenrohr eingeschoben ist, wobei das Innenrohr so dimensioniert ist, dass sein Lumen an den Lumen des zu verbindenden Blutbeutel-Anschlusses angepasst ist, und es im Inneren des Außenrohres ohne Lumenverengung das Ende des Blutbeutel-Anschlusses kontaktiert,
dass getrennt und in Axialrichtung hinter dem ersten Außenrohr das zweite Außenrohr angeordnet ist, in das der Überstand hineinragt und in dem sich das Siegelelement befindet, das in einem auseinander gezogenen Zustand der Außenrohre im Bewegungsbereich des Innenrohrs innerhalb des Lumens des zweiten Außenrohres angeordnet ist und durch Zusammenschieben der beiden Außenrohre nach Kontakt mit dem Innenrohr aufbrechbar ist,
wobei das Siegelelement ein zu zerstörendes Häutchen umfasst und in einem seiner Konfiguration derart angepassten Rezess des zweiten Außenrohrs angeordnet ist, dass im zusammengeschobenen Zustand der Außenrohre im Wesentlichen alle aufgebrochenen Teile des Siegelelementes im Bereich des Rezesses zwischen Innenrohr-Aussenwand und Außenrohr-Innenwand nach außen gedrückt und außerhalb des Fließweges verbleiben.

Bei dem Übergangsverschluss gemäß Erfindung, wie vorstehend bezeichnet, ist als Vorteil anzusehen, dass sich keine Stufe einer Kanüle und keine Reste des abgebrochenen Verschlusses mit scharfen Kanten oder Spitzen ergeben, die in den Fluss des Blutpräparates hinein ragen könnten. Durch den vorbeschriebenen Übergangsverschluss gemäß Erfindung lassen sich die Nachteile der eingangs genannten Art vermeiden, wobei zusätzlich durch das Einschieben nach Art eines Teleskops im zusammengeschobenen Zustand der Außenrohre alle aufgebrochenen Teile des Siegelelementes zwischen Innenrohr-Außenwand und Außenrohr-Innenwand verbleiben und keine Beschädigung der Erythrozyten hervorrufen können.

Als Siegelelement kann ein gesondertes oder aber einstückig mit zweiten Außenrohr gefertigtes Teil verwendet werden, das im Verschlusszustand beispielsweise eine scheibenförmige, hemisphärische (halbkugelförmige) oder konische Konfiguration hat.

Das Siegelelement umfasst ein zu zerstörendes Häutchen, wobei letzteres sich vorzugsweise aus mehreren, durch Sollbruchstellen getrennten Lamellen zusammensetzt. abgerundet verbleiben, um eine Beschädigung von Erythrozyten bei dem nachfolgenden Fließvorgang zu vermeiden.

Weiterhin wird vorgeschlagen, die an ihren Stirnseiten sich gegenüberliegenden Enden der Außenrohre mit einem Rastverschluss zu versehen, so dass die zusammengeschobenen Außenrohre im eng zueinander liegenden Zustand verbleiben, ohne wieder leicht auseinander gezogen werden zu können.

Auch kann wenigstens eines der Außenrohre außenseitig mit Griffmulden oder einem reibungserhöhten Bereich versehen sein, so dass ein leichtes und definiertes Zusammenschieben der beiden Außenrohre ermöglicht ist.

Schließlich kann den hohen Anforderungen an Sterilität bis zur endgültigen Benutzung dadurch Genüge getan werden, dass ein Schubbereich zwischen den Außenrohren von einem Schutzbalg oder einem Schutzrohr umgeben ist.

Die Figuren zeigen im Einzelnen:
- Fig. 1: eine schematisierte Darstellung eines Blutbeutelsystems;
- Fig. 2: einen Übergangsverschluss in auseinandergezogenem, geschlossenem Zustand;
- Fig. 3: einen Übergangsverschluss in zusammengeschobenem, geöffneten Zustand;
- Fig. 4a/4b: ein Siegelelement im geschlossenen bzw. aufgebrochenen Zustand.
- Fig.5: eine andere Ausführungsform von Siegelelement und Außenrohr.

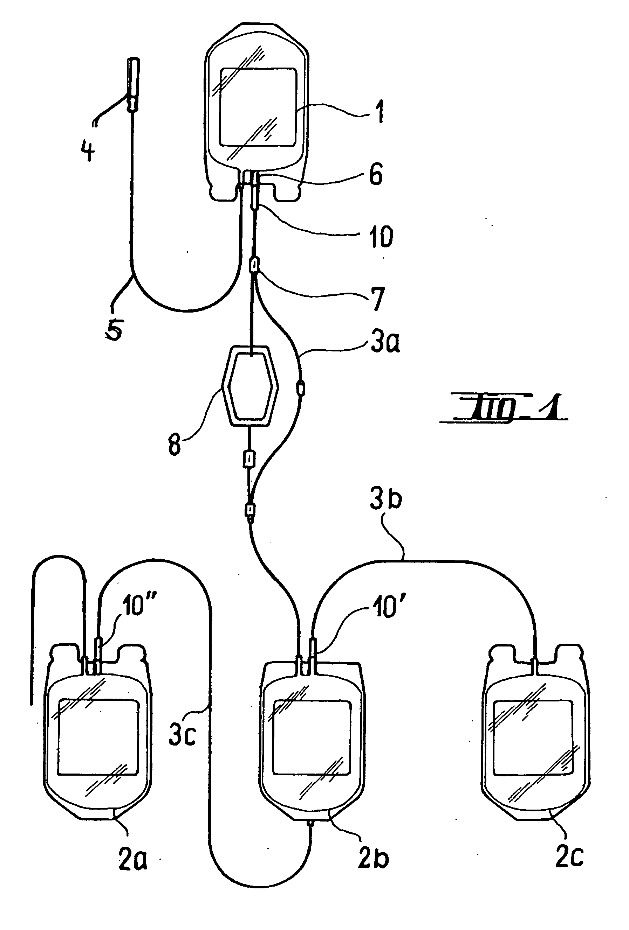

Ein in Figur 1 schematisch dargestelltes Beutelsystem zur Entnahme von Vollblutpräparaten bei Menschen umfasst einen Primärbeutel 1 und drei Sekundärbeutel 2a, 2b und 2c, die verschiedene Funktionen haben. Ausgehend von einer Einheit Vollblut, das bei einer Blutspende in dem Primärbeutel 1 aufgefangen wurde, werden durch Zentrifugier- und Filterschritt Blutplasma, Thrombozyten-Konzentrat (aus Buffy Coat) und ein leukozyten-abgereichertes Erythrozytenkonzentrat gewonnen und in die Sekundärbeutel 2a bis 2c überführt und darin gelagert. Die Beutel 1 bzw. 2, die jeweils etwa 500 cm³ Flüssigkeit aufnehmen können, sind durch Transferschläuche 3a, 3b und 3c verbunden. Als Material für die Einzelteile des Übergangsverschlusses werden Kunststoffe, nämlich insbesondere Polyolefine, Polyvinylchloride und/oder Polycarbonate verwendet, die so temperaturbeständig sein müssen, dass sie durch Dampf sterilisierbar sind. Hieran angepasst ist auch das übrige Material der Beutelsysteme. Wesentlich für alle Ausführungsformen des Übergangsverschlusses ist die Möglichkeit, ihn zusammen mit den Blutbeuteln sterilisieren zu können.

Der Primärbeutel 1 ist mit einer Einsteckkanüle 4 und mit einem Einlassschlauch 5 ausgestattet, durch welche das von einer Person gespendete Blut in den Primärbeutel 1 gelangt. Der Primärbeutel 1 ist mit einem Auslassstutzen 6 in Form eines sich an das Beutelmaterial anschließenden Röhrchens versehen. An den Auslassstutzen 6 schließt sich ein Übergangsverschluss 10 an.

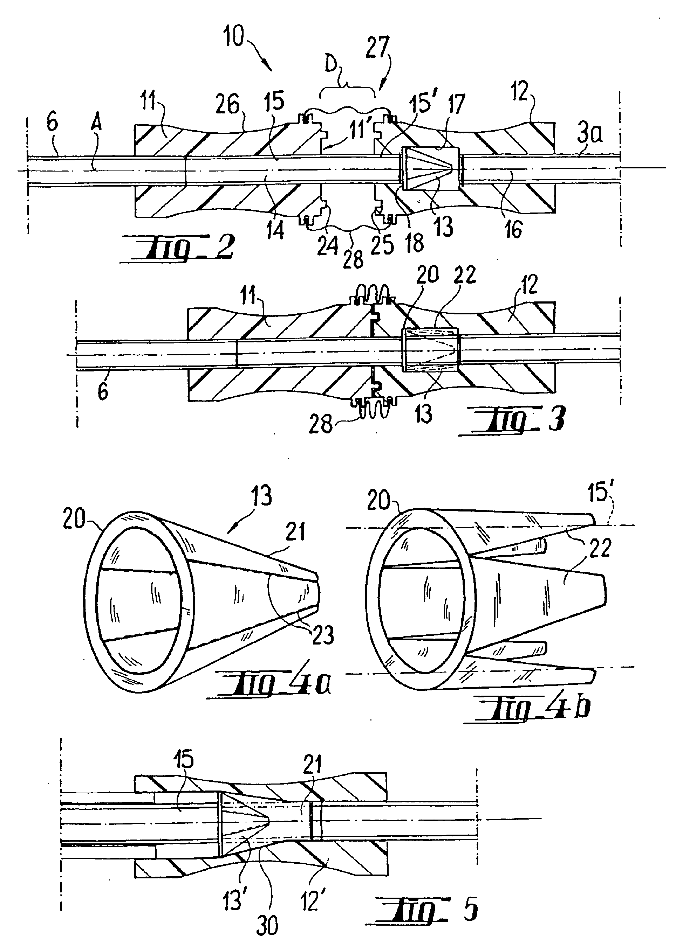

Der Übergangsverschluss gemäß den Figuren 2 und 3 umfasst zwei relativ kurze Außenrohre 11 und 12, die ähnlich wie Spindeln gestaltet sind und von Hand leicht ergriffen werden können. Beide Außenrohre sind mit einer axialen Bohrung 14 beziehungsweise 16 versehen.

Zur Verbindung des Blutbeutel-Ausgangstutzens 6 mit dem ersten Außenrohr 11 ist dieser in die Bohrung 14 auf etwa 1/3 ihrer Länge eingeschoben. Von der anderen Seite ist in die Bohrung 14 ein etwa sechs bis zehn cm langes Innenrohr 15 eingeschoben, wobei dessen Länge so bemessen ist, dass das Innenrohr 15 mit einem Überstand 15' über die Stirnseite 11' des Außenrohrs 11 übersteht und im Inneren des Außenrohrs ohne Lumenveränderung das Ende des Blutbeutel-Ausgangstutzens 6 kontaktiert.

Funktion und detaillierter Aufbau eines Übergangsverschlusses 10 (bzw. 10' und 10") werden anhand der Figuren 2 und 3 erläutert.

Der Übergangsverschluss 10 besteht im Wesentlichen aus vier Teilen, nämlich aus zwei getrennten, in Axialrichtung hintereinander liegenden Außenrohren 11, 12, dem Innenrohr 15 und einem Siegelelement 13. In die Bohrung 14 (in Figur 2 auf der linken Seite) ist vom Ende her der Auslassstutzen 6 eingeführt und dort festgelegt, beispielsweise durch Verklebung. Von der anderen Seite des Außenrohres 11 ist das Innenrohr 15 in die Bohrung 14 eingeschoben und so arretiert, dass das Innenrohr mit seinem Überstand 15' über die Stirnseite 11' des Außenrohres übersteht. Das erste Außenrohr 11 kann mit dem Innenrohr 15 auch einstückig hergestellt sein, wobei in vorteilhafter Weise eine Stufe innerhalb der Bohrung im Bereich des Übergangs von Innenrohr zu Außenrohr entfällt.

Das dem ersten Außenrohr 11 in Abstand D gegenüberliegende zweite Außenrohr 12 ist ebenfalls zylindrisch oder spindelförmig gestaltet und mit einer konzentrisch zur Mittelachse A liegenden Bohrung 16 versehen. Von dem zum Auslassstutzen 6 gegenüberliegenden Ende her ist das Ende eines Transferschlauches 3a eingeführt. Letzterer ist gegenüber dem zweiten Außenrohr fixiert und mit einer Schlauchweiche 7 versehen, so dass der Inhalt wahlweise auch über ein Filterelement 8 geleitet werden kann. Am Ausgang des Sekundärbeutels 2b sowie am Eingang des Sekundärbeutels 2a sind gleichartige Übergangsverschlüsse 10' und 10" eingebaut.

Das zweite Außenrohr 12 weist als Erweiterung der Bohrung 16 im Mittelbereich einen Rezess 17 auf, in dem sich das Siegelelement 13 befindet. Das Außenrohr 12 wird in diesem Fall vorzugsweise aus zwei Rohrschalen hergestellt. Das Siegelelement 13, von dem eine Ausführungsform in den Figuren 4a und 4b dargestellt ist, sperrt zunächst den Fluss durch den Übergangsverschluss 10 (Figur 2). Das Verschieben der Außenrohre 11, 12 gegeneinander für das eigentliche Öffnen wird erleichtert durch Griffmulden 26.

Wie aus der Figur 2 zu erkennen ist, ragt der Überstand 15' des Innenrohrs 15 in die Bohrung 16 des zweiten Außenrohrs 12 hinein. Das Lumen dieser Bohrung 16 ist so dimensioniert, dass das Innenrohr 15 dort axial abgedichtet verschiebbar ist. Das Innenrohr endet vor dem Siegelelement 13.

Zum Öffnen des Übergangsverschlusses werden gemäß Figur 3 die Außenrohre 11 und 12 manuell zusammengeschoben. Dabei stößt der Überstand 15' des Innenrohrs 15 durch das Siegelelement 13 und öffnet dieses, so dass ein Flussweg durch das Siegelelement und durch den Übergangsverschluss insgesamt hergestellt ist.

Dabei ist das Siegelelement 13 so gestaltet und vorgeformt, dass im zusammengeschobenen Zustand der Außenrohre 11 und 12 alle aufgebrochenen Teile 22 des Siegelelementes 13 zwischen der Innenrohr-Außenwand und der Außenrohr-Innenwand verbleiben. Das in den Figuren 4a und 4b dargestellte Siegelelement 13 hat eine konische Form. Die Basis wird von einem Ring 20 gebildet, der am Flansch 18 des Rezesses 17 verklebt ist. An den Ring 20 schließt sich ein zu zerreißendes Häutchen 21 an, das einen Kegelmantel bildet. Der Kegelmantel bei dem dargestellten Ausführungsbeispiel ist in sechs Lamellen 22 aufgeteilt, die im ungetrennten Zustand durch Sollbruchstellen 23 getrennt sind. Wenn die Spitze des Innenrohres 15 den Ring 20 durchstößt, werden die einzelnen Lamellen 22 aufgerissen und durch die Außenseite des Innenrohres abgedeckt. Damit kommen keine scharfkantigen oder abgerissenen Teile des Siegelelementes mit der Blutflüssigkeit in Kontakt.

Da das Siegelelement 13 in einem seiner Konfiguration angepassten Rezess 17 des zweiten Außenrohrs 12 angeordnet ist, werden im zusammengeschobenen Zustand der Außenrohre alle aufgebrochenen Lamellen 22 des Siegelelementes 13 im Bereich des Rezesses zwischen Innenrohr-Außenwand und Außenrohr-Innenwand nach außen gedrückt und verbleiben außerhalb des Fließweges.

Im zusammengeschobenen Öffnungszustand gemäß Figur 3 stoßen die gegenüberliegenden Stirnseiten der Außenrohre 11,12 aneinander. Um ein versehentliches Auseinanderziehen zu verhindern, sind die Stirnseiten 11' mit Rastelementen 24 und 25 versehen, die nach Art eines Druckknopfes eine Rastverbindung herstellen. Stattet man diese Einheit mit einer erforderlichen Mindestlösekraft von etwa 5 N oder mehr aus, wird verhindert, dass ein unbeabsichtigtes Auseinanderziehen eintritt.

Weiterhin ist vorgesehen, den Schubbereich 27, der sich zu beiden Seiten des freien Abstandes D erstreckt, mit einem zylindrischen Schutzbalg 28 zu umgeben. Der Schutzbalg 28 dichtet das Innenrohr 15 und den Eintrittsbereich des Innenrohres ab, so dass nach einer Dampfsterilisation keine Exposition gegen eine nicht-keimfreie Umgebung eintreten kann. Ein solcher Schutzbalg 28 besteht beispielsweise aus einer zick-zack-gefaltenen PVC-Manschette. Anstelle des Balges kann auch ein dichtschliessendes Überzugsrohr verwendet werden, in dem sich die Außenrohre gegeneinander verschieben lassen.

Das in den Figuren 4a/4b dargestellte Siegelelement 13 kann auch abgewandelt werden. Beispielsweise kann es plättchenförmig, das heißt eben gestaltet sein oder eine abgerundete Form (Figur 5) - Bezugszahl 13' - haben. Anstelle eines Rezesses kann auch eine konvergierende Öffnung 30 im zweiten Außenrohr 12' eingeformt sein, wie dies ebenfalls aus Figur 5 erkennbar ist. Das Siegelelement 13' stößt bei dem Durchstoßen durch das Innenrohr 15 gegen die Rohrverengung als Anschlag an. Das Innenrohr 15 wird bis in das verengte Lumen 31 vorgeschoben. Auch hier sind nach dem Zusammenschieben und damit Öffnen des Siegelelementes alle gerissenen Teile hinter dem Innenrohr 15 eingeklemmt, so dass ein ungehinderter Durchfluss möglich ist.

## Patentansprüche

1. Übergangsverschluss (10) für Blutbeutelsysteme, über den ein Blutbeutel-Anschluss (6), der einen bestimmten Lumen aufweist, mit einem Transferschlauch (3a) zu verbinden ist und der unter Sterilhaltung des Fließweges zu öffnen ist, mit folgenden Teilen:
- zwei in Axialrichtung hintereinander liegenden und zueinander axial beweglichen Außenrohren (11, 12), die an ihren entfernt liegenden Enden mit dem Blutbeutel-Anschluss (6) bzw. dem Transferschlauch (3a) verbindbar sind,
- einem Innenrohr (15), das mit dem ersten Außenrohr (11) endseitig überstehend verbunden ist, so dass es mit einem Überstand (15') über die Stirnseite (11') des Außenrohres (11) übersteht, und axial verschiebbar in dem Lumen des zweiten Außenrohres (12) eingeführt ist,
- einem Siegelelement (13), das den Fluss durch den Übergangsverschluss (10) sperrt,
**dadurch gekennzeichnet,**
**dass** zur Verbindung eines Blutbeutel-Anschlusses (6) mit einem Transferschlauch (3) der Blutbeutel-Anschluss (6) von der einen Seite des ersten Außenrohres (11) und von der gegenüberliegenden Seite das Innenrohr (15) eingeschoben ist, wobei das Innenrohr (15) so dimensioniert ist, dass sein Lumen an den Lumen des zu verbindenden Blutbeutel-Anschlusses (6) angepasst ist, und es im Inneren des Außenrohres (11) ohne Lumenverengung das Ende des Blutbeutel-Anschlusses (6) kontaktiert,
**dass** getrennt und in Axialrichtung hinter dem ersten Außenrohr (11) das zweite Außenrohr (12) angeordnet ist, in das der Überstand (15') hineinragt und in dem sich das Siegelelement (13) befindet, das in einem auseinander gezogenen Zustand der Außenrohre (11,12) im Bewegungsbereich des Innenrohrs (15) innerhalb des Lumens des zweiten Außenrohres (12) angeordnet ist und durch Zusammenschieben der beiden Außenrohre (11, 12) nach Kontakt mit dem Innenrohr (15) aufbrechbar ist,
wobei das Siegelelement (13) ein zu zerstörendes Häutchen (21) umfasst und in einem seiner Konfiguration derart angepassten Rezess (17) des zweiten Außenrohrs (12) angeordnet ist, dass im zusammengeschobenen Zustand der Außenrohre im Wesentlichen alle aufgebrochenen Teile (Lamellen 22) des Siegelelementes im Bereich des Rezesses zwischen Innenrohr-Außenwand und Außenrohr-Innenwand nach außen gedrückt und außerhalb des Fließweges verbleiben.

2. Übergangsverschluss nach Anspruch 1, **dadurch gekennzeichnet, dass** das Siegelelement (13) im Verschlusszustand eine scheibenförmige, hemisphärische oder konische Konfiguration hat.

3. Übergangsverschluss nach Anspruch 1 oder 2, **dadurch gekennzeichnet, dass** das Häutchen (21) sich aus mehreren, durch Sollbruchstellen getrennten Lamellen (22) zusammensetzt .

4. Übergangsverschluss nach einem der vorhergehenden Ansprüche, **dadurch gekennzeichnet, dass** das freie Ende des Innenrohrs (15) verjüngt ist.

5. Übergangsverschluss nach einem der vorhergehenden Ansprüche, **dadurch gekennzeichnet, dass** die Stirnseiten der sich gegenüberliegenden Enden der Außenrohre mit einem Rastverschluss (24, 25) versehen sind.

6. Übergangsverschluss nach einem der vorhergehenden Ansprüche, **dadurch gekennzeichnet, dass** wenigstens eines der Außenrohre außenseitig mit Griffmulden (26) oder reibungserhöhten Bereichen versehen ist.

7. Übergangsverschluss nach einem der vorhergehenden Ansprüche, **dadurch gekennzeichnet, dass** ein Schubbereich (27) zwischen den Außenrohren von einem Schutzbalg (28) oder Schutzrohr umgeben ist.

## Claims

1. A transition closure (10) for blood bag systems, via which a blood bag outlet socket (6) that exhibits a certain lumen is to be connected to a transfer hose (3a) and is to be opened while keeping the flow path sterile, having the following parts:
- two outer pipes (11, 12) that lie one behind the other in the axial direction and can be moved relative to each other axially, that can be connected at their remote ends to the blood bag outlet socket (6) or the transfer hose (3 a),
- an inner pipe (15) that is connected to the first outer pipe (11) in a way protruding at the end, such that it protrudes with a protrusion (15') beyond the end face (11') of the outer pipe (11), and is inserted, guidable axially, into the lumen of the second outer pipe (12),
- a sealing element (13) that blocks the flow through the transition closure (10),
**characterized in**
- **that** to connect a blood bag outlet socket (6) to a transfer hose (3), the blood bag outlet socket (6) is inserted from the one side of the first outer pipe (11) and the inner pipe (15) from the opposite side, the inner pipe (15) being dimensioned such that its lumen is matched to the lumen of the connecting blood bag outlet socket (6) and it contacts the end of the blood bag outlet socket (6) inside the outer pipe (11) without lumen contraction,
- **that** separate and in the axial direction behind the first outer pipe (11) the second outer pipe (12) is arranged into which the protrusion (15') projects and in which the sealing element (13) is situated that in an extended state of the outer pipes (11, 12) is arranged inside the motion area of the inner pipe (15) inside the lumen of the second outer pipe (12) and can be broken open by telescoping together the two outer pipes (11, 12) after contacting the inner pipe (15),
- the sealing element (13) comprising a destructible membrane (21) and being arranged in a recess (17) of the second outer pipe (12), the recess being adapted in terms of its configuration such that in the telescoped-together state of the outer pipes essentially all broken-open parts (lamellae 22) of the sealing element are pressed outwards in the area of the recess between inner-pipe outer wall and outer-pipe inner wall and remain outside the flow path.

2. The transition closure according to Claim 1, **characterized in that** in the closed state the sealing element (13) exhibits a disc-shaped, hemispherical or conical configuration.

3. The transition closure according to Claim 1 or 2, **characterized in that** the membrane (21) is composed of several lamellae separated by weak points.

4. The transition closure according to one of the preceding claims, **characterized in that** the free end of the inner pipe (15) is tapered.

5. The transition closure according to one of the preceding claims, **characterized in that** the end faces of the mutually opposite ends of the outer pipes are equipped with a latching closure (24, 25).

6. The transition closure according to one of the preceding claims, **characterized in that** the outside of at least one of the outer pipes is equipped with recessed grips (26) or friction-increasing areas.

7. The transition closure according to one of the preceding claims, **characterized in that** a thrust area (27) between the outer pipes is surrounded by a protective bellows (28) or protective pipe.

## Revendications

1. Fermeture de jonction (10) pour les systèmes pour poches de sang, par lequel se rattache une jonction (6) présentant un certain lumen, au moyen d'un tuyau souple de transfert (3a), et qui doit être ouvert tout en maintenant stérile le conduit d'écoulement, avec les pièces suivantes :
- deux tubes extérieurs, situés l'un derrière l'autre et s'avançant l'un vers l'autre dans un déplacement axial (11, 12), qui peuvent se raccorder aux extrémités lointaines avec la jonction de la poche de sang (6) ou avec le tuyau souple de transfert,
- un tube intérieur (15) lié au premier tube extérieur (11) au niveau de l'extrémité, et en saillie, de sorte qu'il dépasse (15') de la partie frontale (11') du tube extérieur (11), et qu'il est inséré dans le lumen du deuxième tube extérieur et peut coulisser en direction axiale,
- un élément de scellement (13), bloquant l'écoulement au moyen de la fermeture de jonction (10),
**caractérisée en ce que**
pour le raccordement de la jonction de la poche de sang (6) à un tuyau souple de transfert (3), la jonction de la poche de sang (6) est insérée sur un côté du premier tube extérieur (11), et sur le côté opposé du tube intérieur (15), à savoir que le tube intérieur (15) est dimensionné de manière à ce que son lumen soit ajusté au lumen de la jonction de la poche de sang (6) à raccorder, et qu'il soit en contact avec l'extrémité de la jonction de la poche de sang (6) à l'intérieur du tube extérieur (11), sans que le lumen devienne plus étroit,
le deuxième tube extérieur (12) se trouve, séparément et en direction axiale derrière le premier tube extérieur (11), à savoir que dans ce deuxième tube extérieur (12), dans lequel se trouve la saillie (15') et l'élément de scellement (13), situé, lorsque les tubes extérieurs (11,12) sont déployés, dans la zone de de mouvement du tube intérieur (15), à l'intérieur du lumen du deuxième tube extérieur (12), et qu'en faisant coulisser les deux tubes extérieurs (11,12) peut être brisé après un contact avec le tube intérieur (15), à savoir que l'élément de scellement (13) entoure une pellicule à détruire (21) et est situé dans un renfoncement (17) adapté à sa configuration qui, lorsque les tubes extérieurs sont repliés, toutes les pièces rompues (lamelles 22) de l'élément de scellement, au niveau du renfoncement entre la paroi extérieure du tube intérieur et la paroi intérieure du tube extérieur restent pressés vers l'extérieur et hors du conduit d'écoulement.

2. Fermeture de jonction selon la revendication 1, **caractérisée en ce que** l'élément de scellement (13), lorsqu'il est fermé, présente une configuration discoïde, hémisphérique ou conique.

3. Fermeture de jonction selon la revendication 1 ou 2, **caractérisée en ce que** la pellicule (21) se compose de plusieurs lamelles (22) séparées par des points de rupture prévus à cet effet.

4. Fermeture de jonction selon l'une des revendications précédentes, **caractérisée en ce que** l'extrémité du tuyau intérieur (15) soit rajeunie.

5. Fermeture de jonction selon l'une des revendications précédentes, **caractérisée en ce que** les parties frontales des extrémités se faisant face des tuyaux extérieurs sont pourvues d'une grille-lock (24-25).

6. Fermeture de jonction selon l'une des revendications précédentes, **caractérisée en ce qu'**au moins un des tuyaux extérieurs soit, à l'extérieur, avec

7. Fermeture de jonction selon l'une des revendications précédentes, **caractérisée en ce qu'**une zone de poussée (27) soit prévue entre les tuyaux extérieurs d'une poutre protectrice (28) ou entourée d'un tuyau protecteur.
